# EUROPÄISCHE PATENTSCHRIFT

(11) **EP 2 768 474 B1**
(45) Veröffentlichungstag und Bekanntmachung des Hinweises auf die Patenterteilung: **26.04.2017**
(21) Anmeldenummer: 12772783.2
(22) Anmeldetag: 12.10.2012
(51) Int. Cl.: A61K 8/37, A61K 8/46, A61Q 5/02, A61Q 19/10

(54) **MILDES KOSMETISCHES REINIGUNGSMITTEL**
MILD COSMETIC CLEANSING COMPOSITION
NETTOYANT COSMÉTIQUE DOUX

(30) Priorität: 20.10.2011 DE 102011084888
(43) Veröffentlichungstag der Anmeldung: 27.08.2014
(73) Patentinhaber: Henkel AG & Co. KGaA, 40589 Düsseldorf (DE)
(72) Erfinder: FAHL, Madlen, 21442 Tangendorf (DE); HENTRICH, Dirk, 22457 Hamburg (DE); SCHRÖDER, Thomas, 22395 Hamburg (DE); MEYER, Jens, 22880 Wedel (DE)
(86) Internationale Anmeldenummer: PCT/EP2012/070249
(87) Internationale Veröffentlichungsnummer: WO 2013/057045

(56) Entgegenhaltungen:
- EP-A1- 1 674 132
- EP-A1- 2 468 842
- EP-A2- 0 569 028
- WO-A1-2009/135007
- WO-A2-2009/063250
- WO-A2-2011/007174
- WO-A2-2011/015857
- WO-A2-2011/015858

## Beschreibung

Die Erfindung liegt auf dem Gebiet der Kosmetik und betrifft milde Reinigungsmittel, die eine spezielle Tensidmischung enthalten.

Kosmetische Reinigungsmittel, wie beispielsweise Haarshampoos, basieren auf klassischen anionischen, amphoteren, zwitterionischen, nichtionischen und/oder kationischen Tensiden.
Aufgrund ihres hervorragenden Reinigungs- und Schaumvermögens werden überwiegend anionische Tenside, gegebenenfalls in der Mischung mit geringen Mengen an Co-Tensiden, eingesetzt.
Typische anionische Tenside, die in einer Vielzahl im Handel erhältlicher Shampoos eingesetzt werden, sind Alkylsulfate oder Alkylethersulfate.
Alkylethersulfate werden üblicherweise bevorzugt, da sie milder sind und über ein ausgezeichnetes Schaumvermögen verfügen.

Bei der Formulierung besonders milder Reinigungszusammensetzungen für die Anwendung auf sensiblen Hautpartien (wie beispielsweise der Gesichtshaut) oder für die Anwendung auf Babyhaut sind Alkylethersulfate nicht immer zufriedenstellend, denn sie weisen ein für diese Anwendungen zu hohes Reizpotential auf.

In der Vergangenheit wurden daher zahlreiche Versuche unternommen besonders milde Tensidmischungen zu finden, die ausreichend hohe Schaummengen und - qualitäten aufweisen, und die kein oder nur ein geringes Reizpotential auf der Haut und/oder der Schleimhaut aufweisen.

In der Anmeldung WO 92/084440 werden milde Tensidmischungen mit hervorragenden Schaumeigenschaften offenbart, die eine Mischung aus Acylisethionaten, zwitterionischen Tensiden und Alkylethersulfaten enthalten.

WO 11/015857 offenbart Reinigungszusammensetzungen mit niedrigem Irritationspotential, die neuartige C₅₋₃₀-Alkoyl-Alkyl-Isethionate und amphotere Tenside in einem Gewichtsverhältnis von 4 : 1 bis 1 : 4 enthalten. Die milden Reinigungsmittel eignen sich für eine Anwendung als Baby-Shampoo.

In der Anmeldung WO 2011/15858 werden milde Tensidmischungen für die Anwendung auf besonders sensibler Haut und/oder Babyhaut offenbart. Die Tensidmischungen umfassen anionische Isethionat-Tenside (a), amphotere Tenside (b) und alkoxylierte nichtionische Tenside (c) in Gewichtsverhältnissen a : b von 0,5 : 1 bis 2 : 1 und c : (a + b) von < 1,2 : 1.

In den Dokumenten EP 1674132, EP 569028 und WO 2009/135007 werden weitere (ternäre) Tensidmischungen offenbart, die u.a. Alkyl(oligo)glucoside, Aminoxide und Glycerylester als besonders milde Bestandteile enthalten.

Nachteilig an einer Vielzahl milder Haut- und Haarreinigungsmittel ist, dass deren bessere Hautverträglichkeit oftmals zulasten der Textur der Reinigungsmittel geht.
Weiterhin konnte beobachtet werden, dass die Pflegeeigenschaften milder Reinigungsmittel (insbesondere auf den Haaren) nicht immer zufriedenstellend sind.

Um die Pflegeeigenschaften der Mittel zu verbessern, wurden milden Tensidmischungen in der Praxis oftmals Silikone und/oder ethoxylierte Komponenten zugesetzt, was aus anwendungstechnischer Sicht in sensitiven Produkten und/oder Baby-Pflegeprodukten nicht erwünscht ist.

Der vorliegenden Erfindung lag die Aufgabe zugrunde, besonders milde kosmetische Reinigungsmittel herzustellen, die gut haut- und schleimhautverträglich sind.
Die Reinigungsmittel sollten eine anwenderfreundliche Textur aufweisen und fließfähig sein.
Weiterhin sollten die Pflegeeigenschaften milder Reinigungszusammensetzungen ohne einen Zusatz ethoxylierter Komponenten und/oder einen Zusatz von Silikonen verbessert werden. Insbesondere sollten milde kosmetische Reinigungsmittel entwickelt werden, die die zuvor genannten Anforderungen auch bei niedrigeren pH-Werten erfüllen.

Gegenstand der Erfindung ist ein mildes kosmetisches Reinigungsmittel, das in einem geeigneten Träger
a) 1 bis 6 Gew.-% mindestens eines anionischen Tensids der nachfolgenden Formel (I), 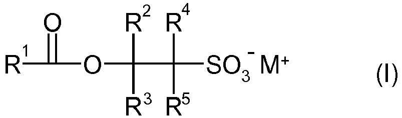 in der
   - R¹ für einen linearen oder verzweigten, gesättigten oder ungesättigten Alkylrest mit 6 bis 30 Kohlenstoffatomen steht,
   - mindestens einer der Reste R² bis R⁵ für einen C₁-C₄-Alkylrest, und die anderen Reste unabhängig voneinander für ein Wasserstoffatom oder einen C₁-C₄-Alkylrest stehen, und
   - M⁺ für ein Ammonium-, ein Alkanolammonium oder ein Metallkation steht,
b) 2 bis 10 Gew.-% mindestens eines amphoteren und/oder zwitterionischen Tensids und
c) 0,5 bis 10 Gew.-% mindestens eines nichtionischen Tensids und/oder mindestens eines nichtionischen Emulgators enthält, der ausgewählt ist aus mindestens einer der nachfolgenden Gruppen der
   - Alkyl(oligo)glycoside,
   - Aminoxide und/oder
   - Glycerinmono- und/oder -di-(C₆-C₂₄-)Carbonsäureester enthält,
wobei sich die Mengenangaben auf das Gesamtgewicht des Reinigungsmittels beziehen, und wobei es weniger als 0,5 Gew.-%, mehr bevorzugt weniger als 0,25 Gew.-% und besonders bevorzugt weniger als 0,1 Gew.-% ethoxylierte Wirkstoffe enthält.

Unter einem geeigneten Träger wird bevorzugt ein wässriger oder wässrig-alkoholischer Träger verstanden.
Bevorzugt enthält der Träger mindestens 50 Gew.-%, mehr bevorzugt mindestens 60 Gew.-% und besonders bevorzugt mindestens 70 Gew.-% Wasser.
Weiterhin kann der kosmetische Träger 0,01 bis 40 Gew.-%, bevorzugt 0,05 bis 35 Gew.-% und insbesondere 0,1 bis 30 Gew.-% mindestens eines Alkohols enthalten, der ausgewählt sein kann aus Ethanol, 1-Propanol, 2-Propanol, Isopropanol, Glycerin, Diglycerin, Triglycerin, 1-Butanol, 2-Butanol, 1,2-Butandiol, 1,3-Butandiol, 1-Pentanol, 2-Pentanol, 1, Hexanol, 2-Hexanol, Sorbitol, Sorbitan, Benzylalkohol, Phenoxyethanol oder Mischungen dieser Alkohole.
Bevorzugt sind die wasserlöslichen Alkohole.
Besonders bevorzugt sind Ethanol, 1-Propanol, 2-Propanol, Isopropanol, Glycerin, Benzylalkohol und/oder Phenoxyethanol sowie Mischungen dieser Alkohole. Insbesondere bevorzugt ist Glycerin.

Zur Erzielung optimaler Milde und Pflege enthalten erfindungsgemäße Reinigungsmittel die Tenside/Emulgatoren a), b) und c) in einem Gewichtsverhältnis von (1-2): (2-5): (1-3).

Bevorzugte anionische Tenside der zuvor genannten Formel (I) weisen als Rest R¹ einen linearen oder verzweigten, gesättigten oder ungesättigten Alkylrest mit 8 bis 18 Kohlenstoffatomen auf. Besonders bevorzugt steht der Rest R¹ für einen C₈-, C₁₀-, C₁₂-, C₁₄-, C₁₆-Rest oder für Gemische dieser Fettsäurereste wie sie erhalten werden, wenn sich die Fettsäure(n) aus natürlichen Ölen wie beispielsweise Kokosöl ableitet(n).

In weiterhin bevorzugten anionischen Tensiden der zuvor genannten Formel (I) können die Reste R² bis R⁵ jeweils für eine Methyl-, Ethyl-, n-Propyl-, n-Butyl- oder 2-Butylgruppe stehen.
Bevorzugt steht mindestens einer der Reste für R² bis R⁵ für eine Methyl-, Ethyl- oder eine n-Propylgruppe und insbesondere für eine Methylgruppe.
In einer besonders bevorzugten Ausführungsform steht nur einer der Reste R² bis R⁵ für eine C₁-C₄-Alkylgruppe - insbesondere eine Methylgruppe - und die anderen Restejeweils für ein Wasserstoffatom. Prinzipiell ist es auch möglich, dass das anionische Tensid nach Formel (I) ein Isomerengemisch enthält, in dem sowohl Komponenten enthalten sind, die beispielsweise als Rest R² eine C₁-C₄-Wasserstoffatom aufweisen, als auch Komponenten, die beispielsweise als Rest R⁵ eine C₁-C₄-Alkylgruppe - insbesondere eine Methylgruppe - und als Reste R² bis R⁴ jeweils ein Wasserstoffatom aufweisen.

M⁺ in der zuvor genannten Formel (I) steht bevorzugt für ein Alkalimetallkation oder ein Ammoniumion.
Besonders bevorzugt steht der M⁺ für ein Kalium- oder ein Natriumion und insbesondere bevorzugt für ein Natriumion.

Ganz besonders bevorzugte anionische Tenside der zuvor genannten Formel (I) sind die unter den INCI-Bezeichnungen Natrium Cocoyl Methyl Isethionate, Kalium Cocoyl Methyl Isethionate, Ammonium Cocoyl Methyl Isethionate, Natrium Lauroyl Methyl Isethionate, Kalium Lauroyl Methyl Isethionate, Ammonium Lauroyl Methyl Isethionate, Natrium Myristoyl Methyl Isethionate, Kalium Myristoyl Methyl Isethionate und Ammonium Myristoyl Methyl Isethionate bekannten Verbindungen.

Insbesondere bevorzugt sind Natrium Cocoyl Methyl Isethionate und/oder Natrium Lauroyl Methyl Isethionate. Entsprechende Handelsprodukte sind beispielsweise von der Firma Innospec unter der Handelsbezeichnung "Iselux^{®} LQ-CLR-SB" erhältlich.

Das mindestens eine anionische Tensid der zuvor genannten Formel (I) wird in den erfindungsgemäßen milden Reinigungsmitteln bevorzugt in einer Menge von 1,1 bis 5 Gew.-%, mehr bevorzugt von 1,2 bis 4 Gew.-% und insbesondere von 1,25 bis 3 Gew.-% eingesetzt, wobei sich die Mengenangaben auf das Gesamtgewicht des Reinigungsmittels beziehen.

Geeignete amphotere und/oder zwitterionische Tenside, die in den erfindungsgemäßen Reinigungsmitteln eingesetzt werden können, entsprechen bevorzugt mindestens einer Verbindung der nachfolgenden Formeln (i) bis (vii), in denen der Rest R für einen geradkettigen oder verzweigten, gesättigten oder ein- oder mehrfach ungesättigten Alkyl- oder Alkenylrest mit 7 bis 23 Kohlenstoffatomen (Formeln (i) und (ii)) oder für einen geradkettigen oder verzweigten, gesättigten oder ein- oder mehrfach ungesättigten Alkyl- oder Alkenylrest mit 8 bis 24 Kohlenstoffatomen (Formeln (iii) bis (vii)) steht:

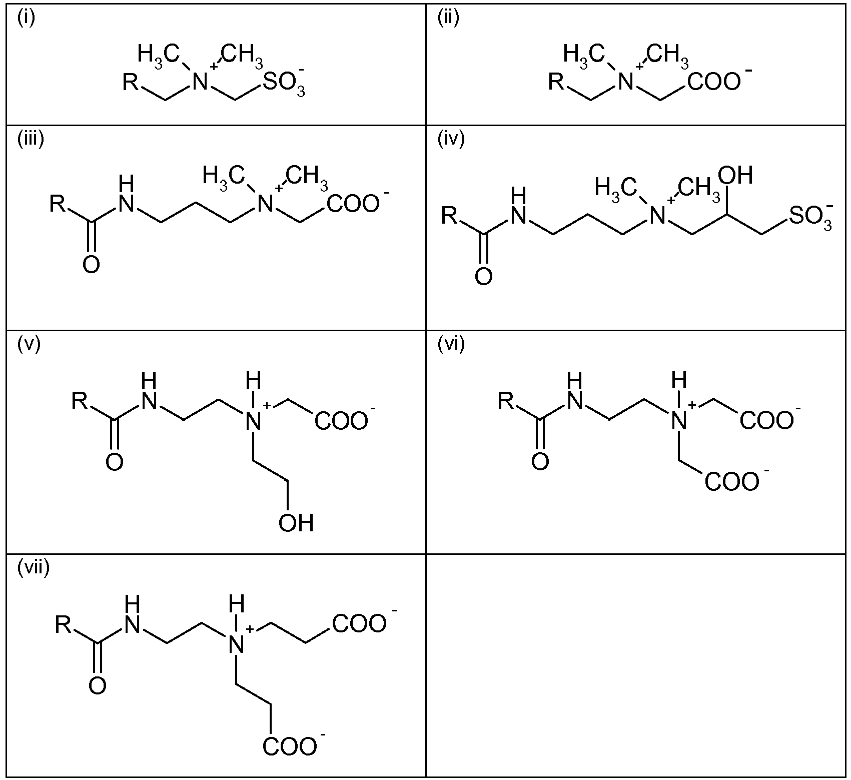

Bevorzugte amphotere und/oder zwitterionische Tenside einer der zuvor genannten Formeln (i) bis (vii) enthalten als Rest R überwiegend einen geradkettigen oder verzweigten, gesättigten, ein- oder mehrfach ungesättigten Alkylrest mit 8 bis 20, mehr bevorzugt von 8 bis 16 und insbesondere mit 8 bis 12 C-Atomen.
Mehr bevorzugt sind amphotere und/oder zwitterionische Tenside, bei denen sich der Rest R von Kokosfett ableitet.
Besonders bevorzugt sind amphotere und/oder zwitterionische Tenside der Formeln (iii), (v), (vi) und (vii).
Insbesondere bevorzugt sind die unter den INCI-Bezeichnungen Cocamidopropylbetain und/oder Cocoampho(di)acetate bekannten und im Handel von mehreren Anbietern erhältlichen amphoteren Tenside.

Das oder die amphoteren/zwitterionischen Tenside einer der zuvor genannten Formeln (i) bis (vii) kann (können) in den erfindungsgemäßen Reinigungsmitteln - bezogen auf deren Gesamtgewicht - bevorzugt in Mengen von 2,5 bis 8,5 Gew.-%, bevorzugt von 3 bis 7,5 Gew.-% und insbesondere von 4 bis 6 Gew.-% eingesetzt werden.

Zu den geeigneten nichtionischen Tensiden und/oder Emulgatoren zählen die
- Alkyl(oligo)glycoside,
- Aminoxide und/oder
- Glycerinmono- und/oder -di-(C₆-C₂₄-)Carbonsäureester.

Geeignete Alkyl(oligo)glycoside können ausgewählt sein aus Verbindungen der allgemeinen Formel der RO-[G]ₓ, in denen sich [G] bevorzugt von Aldosen und/oder Ketosen mit 5-6 Kohlenstoffatomen, vorzugsweise von Glucose ableitet.
Die Indexzahl x steht für den Oligomerisierungsgrad (DP), d.h. für die Verteilung der Mono- und Oligoglycoside. Die Indexzahl x weist vorzugsweise einen Wert im Bereich von 1 bis 10, besonders bevorzugt im Bereich von 1 bis 3 auf, wobei es sich dabei um keine ganze Zahl, sondern um eine gebrochene Zahl handeln kann, die analytisch ermittelt werden kann.
Besonders bevorzugte Alkyl(oligo)glycoside weisen einen Oligomerisierungsgrad zwischen 1,2 und 1,5 auf.
Der Rest R steht bevorzugt für mindestens einen Alkyl- und/oder Alkenylrest mit 4 bis 24 C-Atomen.

Geeignete Aminoxide können ausgewählt sein aus mindestens einer Verbindung der allgemeinen Formeln (II) oder (III)

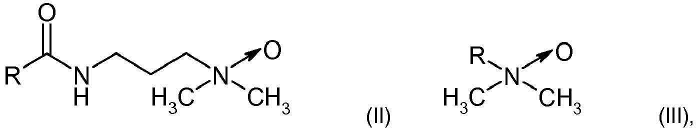

in denen R jeweils für einen geradkettigen oder verzweigten, gesättigten oder ein- oder mehrfach ungesättigten Alkyl- oder Alkenylrest mit 6 bis 24 Kohlenstoffatomen, bevorzugt mit 8 bis 18 Kohlenstoffatomen steht.
Insbesondere bevorzugt sind die unter den INCI-Bezeichnungen Cocamine Oxide, Lauramine Oxide und/oder Cocamidopropylaminoxid bekannten und im Handel von verschiedenen Anbietern erhältlichen Tenside der zuvor genannten Formel (II) oder (III).

Unter geeigneten Glycerinmono- und/oder -di-(C₆-C₂₄-)Carbonsäureestern ist bevorzugt eine unter der INCI-Bezeichnung Glyceryl Caprylate/Caprate bekannte Verbindung zu verstehen. Besonders bevorzugt enthalten die kosmetischen Reinigungsmittel als nichtionisches Tensid und/oder nichtionischen Emulgator mindestens ein Alkyl(oligo)glycosid der allgemeinen Formel RO-[G]ₓ, in der R für einen Alkyl- und/oder Alkenylrest mit 4 bis 22 C-Atomen, G für einen Zuckerrest mit 5 oder 6 C-Atomen und x für Zahlen von 1 bis 10 steht.
Insbesondere bevorzugte Alkyl(oligo)glycoside sind die unter den INCI-Bezeichnungen Caprylyl/Capryl Glucoside, Decyl Glucoside, Lauryl Glucoside und Coco Glucoside bekannten Verbindungen.

Das oder die nichtionischen Tenside und/oder Emulgatoren kann (können) in den erfindungsgemäßen Reinigungsmitteln - bezogen auf deren Gesamtgewicht - bevorzugt in Mengen von 0,75 bis 8 Gew.-%, besonders bevorzugt von 1 bis 6 Gew.-% und insbesondere von 1,5 bis 5 Gew.-% eingesetzt werden.

In einer ersten besonders bevorzugten Ausführungsform enthalten erfindungsgemäße milde kosmetische Reinigungsmittel - bezogen auf ihr Gesamtgewicht -
a) 1,1 bis 5 Gew.-% mindestens eines anionischen Tensids der zuvor genannten Formel (I), in der der Rest R¹ für einen linearen oder verzweigten, gesättigten oder ungesättigten Alkylrest mit 8 bis 18 Kohlenstoffatomen steht, mindestens einer der Reste R² bis R⁵ für einen Methylrest und die anderen Reste jeweils für ein Wasserstoffatom stehen, und M⁺ für ein Alkalimetallkation oder ein Ammoniumion steht,
b) 2,5 bis 8,5 Gew.-% mindestens eines amphoteren und/oder zwitterionischen Tensids einer der zuvor genannten allgemeinen Formeln (iii), (v), (vi) oder (vii), und
c) 0,75 bis 8 Gew.-% mindestens eines nichtionisches Tensids und/oder nichtionischen Emulgators einer der zuvor genannten allgemeinen Formeln (II), (III) oder RO-[G]ₓ.

Innerhalb dieser Ausführungsform ist es mehr bevorzugt, wenn die erfindungsgemäßen Reinigungsmittel - bezogen auf ihr Gesamtgewicht -
a) 1,2 bis 4 Gew.-% mindestens einer der unter den INCI-Bezeichnungen Natrium Cocoyl Methyl Isethionate, Kalium Cocoyl Methyl Isethionate, Ammonium Cocoyl Methyl Isethionate, Natrium Lauroyl Methyl Isethionate, Kalium Lauroyl Methyl Isethionate, Ammonium Lauroyl Methyl Isethionate, Natrium Myristoyl Methyl Isethionate, Kalium Myristoyl Methyl Isethionate und Ammonium Myristoyl Methyl Isethionate bekannten Verbindungen,
b) 3 bis 7,5 Gew.-% mindestens eines der unter den INCI-Bezeichnungen Cocamidopropylbetain und/oder Cocoampho(di)acetate bekannten amphoteren und/oder zwitterionischen Tenside, und
c) 1 bis 6 Gew.-% mindestens eines Alkyl(oligo)glycosids der allgemeinen Formel RO-[G]ₓ enthält, in der R für einen Alkyl- und/oder Alkenylrest mit 4 bis 22 C-Atomen, G für einen Zuckerrest mit 5 oder 6 C-Atomen und x für Zahlen von 1 bis 10 steht.

Insbesondere bevorzugte milde kosmetische Reinigungsmittel dieser Ausführungsform enthalten - bezogen auf ihr Gesamtgewicht -
a) 1,25 bis 3 Gew.-% mindestens einer der unter den INCI-Bezeichnungen Natrium Cocoyl Methyl Isethionate und/oder Natrium Lauroyl Methyl Isethionate bekannten Verbindungen,
b) 4 bis 6 Gew.-% mindestens eines unter der INCI-Bezeichnung Cocamidopropylbetain bekannten amphoteren Tensids, und
c) 1,5 bis 5 Gew.-% mindestens einer der unter den INCI-Bezeichnungen Caprylyl/Capryl Glucoside, Decyl Glucoside, Lauryl Glucoside und Coco Glucoside bekannten Verbindungen.

Zur weiteren Steigerung der pflegenden Eigenschaften können die erfindungsgemäßen milden Reinigungsmittel weiterhin mindestens einen konditionierenden Wirkstoff enthalten, der ausgewählt sein kann aus der Gruppe der
- Proteinhydrolysate,
- kationischen Polymere,
- Vitamine,
- pflanzlichen Öle,
- Glycerin.

Unter geeigneten Proteinhydrolysaten sind Produktgemische zu verstehen, die durch sauer, basisch oder enzymatisch katalysierten Abbau von Proteinen (Eiweißen) erhalten werden können.
Es können Proteinhydrolysate pflanzlichen, tierischen und/oder marinen Ursprungs eingesetzt werden.
Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben.
Bevorzugt sind Proteinhydrolysate pflanzlichen Ursprungs, z. B. Soja-, Mandel-, Reis-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex) und Crotein^{®} (Croda) erhältlich.
Einsetzbar sind auch kationisierte Proteinhydrolysate, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder von biotechnologisch gewonnenen Proteinhydrolysaten, stammen kann. Die den kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die kationischen Proteinhydrolysate und - derivate seien die unter den INCI - Bezeichnungen bekannten und im Handel erhältlichen Produkte genannt: Cocodimonium Hydroxypropyl Hydrolyzed Collagen, Cocodimopnium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed keratin, Hydroxypropyltrimonium Hydrolyzed Rice Bran Protein, Hydroxyproypltrimonium Hydrolyzed Silk, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Silk, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Silk, Quaternium-79 Hydrolyzed Soy Protein, Quaternium-79 Hydrolyzed Wheat Protein.

Das oder die Proteinhydrolysate kann (können) in den erfindungsgemäßen Reinigungsmitteln - bezogen auf deren Gesamtgewicht - bevorzugt in Mengen von 0,01 bis 5 Gew.-%, mehr bevorzugt von 0,025 bis 3 Gew.-% und insbesondere von 0,05 bis 2 Gew.-% eingesetzt werden.

Geeignete kationische Polymere sind beispielsweise:
- quaternisierte Cellulose-Derivate, wie sie unter den Bezeichnungen Celquat^{®} und Polymer JR^{®} im Handel erhältlich sind,
- hydrophob modifizierte Cellulosederivate, beispielsweise die unter dem Handelsnamen SoftCat^{®} vertriebenen kationischen Polymere,
- kationische Alkylpolyglycoside,
- kationiserter Honig, beispielsweise das Handelsprodukt Honeyquat^{®} 50,
- kationische Guar-Derivate, wie insbesondere die unter den Handelsnamen Cosmedia^{®}Guar N-Hance^{®} und Jaguar^{®} vertriebenen Produkte,
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Be zeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
   sowie die unter den Bezeichnungen
- Polyquaternium 2, Polyquaternium 17, Polyquaternium 18, Polyquaternium-24, Polyquaternium 27, Polyquaternium-32, Polyquaternium-37, Polyquaternium 74 und Polyquaternium 89 bekannten Polymere.

Bevorzugte kationische Polymere sind quaternisierte Cellulosepolymere, hydrophob modifizierte kationische Cellulosederivate, kationische Guarderivate und/oder kationische Polymere auf Acrylsäure(derivat)basis, die besonders bevorzugt ausgewählt sind aus den unter den INCI-Bezeichnungen bekannten Polymeren Guar Hydroxypropyltrimonium Chloride, Polyquaternium-6, Polyquaternium-7, Polyquaternium-10, Polyquaternium-37 und/oder Polyquaternium-67. Insbesondere bevorzugt sind die unter den INCI-Bezeichnungen bekannten Polymere Guar Hydroxypropyltrimonium Chloride, Polyquaternium-10 und/oder Polyquaternium-67.

Das oder die kationische(n) Polymer(e), kann (können) in den erfindungsgemäßen Reinigungsmitteln - bezogen auf deren Gesamtgewicht - bevorzugt in einer Menge 0,01 bis 5 Gew.-%, mehr bevorzugt von 0,025 bis 4 Gew.-%, besonders bevorzugt von 0,05 bis 3 Gew.-% und insbesondere von 0,1 bis 2 Gew.-% eingesetzt werden.

Unter geeigneten Vitaminen sind bevorzugt die folgenden Vitamine, Provitamine und Vitaminvorstufen sowie deren Derivate zu verstehen:
- Vitamin A: zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen beispielsweise Vitamin A-Säure und deren Ester, Vitamin A-Aldehyd und Vitamin A-Alkohol sowie dessen Ester wie das Palmitat und das Acetat in Betracht.
- Vitamin B: zur Vitamin B-Gruppe oder zu dem Vitamin B-Komplex gehören u. a.
   ➢ Vitamin B₁ (Thiamin)
   ➢ Vitamin B₂ (Riboflavin)
   ➢ Vitamin B₃. Unter dieser Bezeichnung werden häufig die Verbindungen Nicotinsäure und Nicotinsäureamid (Niacinamid) geführt.
   ➢ Vitamin B₅ (Pantothensäure und Panthenol). Im Rahmen dieser Gruppe wird bevorzugt das Panthenol eingesetzt. Einsetzbare Derivate des Panthenols sind insbesondere die Ester und Ether des Panthenols, Pantolacton sowie kationisch derivatisierte Panthenole. Einzelne Vertreter sind beispielsweise das Panthenoltriacetat, der Panthenolmonoethylether und dessen Monoacetat sowie kationische Panthenolderivate.
   ➢ Vitamin B₆ (Pyridoxin sowie Pyridoxamin und Pyridoxal).
- Vitamin C (Ascorbinsäure): die Verwendung in Form des Palmitinsäureesters, der Glucoside oder Phosphate kann bevorzugt sein. Die Verwendung in Kombination mit Tocopherolen kann ebenfalls bevorzugt sein.
- Vitamin E (Tocopherole, insbesondere α-Tocopherol).
- Vitamin F: unter dem Begriff "Vitamin F" werden üblicherweise essentielle Fettsäuren, insbesondere Linolsäure, Linolensäure und Arachidonsäure, verstanden.
- Vitamin H: Als Vitamin H wird die Verbindung (3aS,4S, 6aR)-2-Oxohexahydrothienol[3,4-d]-imidazol-4-valeriansäure bezeichnet, für die sich aber zwischenzeitlich der Trivialname Biotin durchgesetzt hat.

Die erfindungsgemäßen Reinigungsmittel können bevorzugt Vitamine, Provitamine und Vitaminvorstufen aus den Gruppen A, B, E und H enthalten.
Insbesondere bevorzugt sind Nicotinsäureamid, Biotin, Pantolacton und/oder Panthenol.

Vitamine, Vitaminderivate und/oder Vitaminvorstufen können in den erfindungsgemäßen Reinigungsmitteln - bezogen auf deren Gesamtgewicht - bevorzugt in einer Menge von 0,001 bis 2 Gew.-%, besonders bevorzugt von 0,005 bis 1 Gew.-% und insbesondere von 0,01 bis 0,5 Gew.-% eingesetzt werden.

Unter geeigneten natürlichen (pflanzlichen) Ölen werden üblicherweise Triglyceride und Mischungen von Triglyceriden verstanden. Bevorzugte natürliche Öle sind Kokosnussöl, (süßes) Mandelöl, Walnussöl, Pfirsichkernöl, Aprikosenkernöl, Avocadoöl, Teebaumöl (Tea Tree Oil), Sojaöl, Sesamöl, Sonnenblumenöl, Tsubakiöl, Nachtkerzenöl, Reiskleieöl, Palmkernöl, Mangokernöl, Wiesenschaumkrautöl, Distelöl, Macadamianussöl, Traubenkernöl, Amaranthsamenöl, Arganöl, Bambusöl, Olivenöl, Weizenkeimöl, Kürbiskernöl, Malvenöl, Haselnussöl, Safloröl, Canolaöl, Sasanquaöl, Jojobaöl, Rambutanöl, Kakaoabutter und/oder Shea-Butter.

Pflanzliche Öle können in den erfindungsgemäßen Reinigungsmitteln bevorzugt in Mengen von 0,01 bis 5 Gew.-%, mehr bevorzugt von 0,025 bis 4 Gew.-%, besonders bevorzugt von 0,05 bis 3 Gew.-% und insbesondere von 0,1 bis 2 Gew.-% eingesetzt werden, wobei sich die Mengenangaben auf das Gesamtgewicht der Reinigungsmittel beziehen.

Glycerin kann den erfindungsgemäßen Reinigungsmitteln separat in einer Menge von bis zu 10 Gew.-% (bezogen auf das Gesamtgewicht des Reinigungsmittels) zugegeben werden. Es kann aber auch Bestandteil des wässrig-alkoholischen Trägers sein.

In einer zweiten besonders bevorzugten Ausführungsform enthalten erfindungsgemäße milde kosmetische Reinigungsmittel - bezogen auf ihr Gesamtgewicht -
a) 1,1 bis 5 Gew.-% mindestens eines anionischen Tensids der zuvor genannten Formel (I), in der der Rest R¹ für einen linearen oder verzweigten, gesättigten oder ungesättigten Alkylrest mit 8 bis 18 Kohlenstoffatomen steht, mindestens einer der Reste R² bis R⁵ für einen Methylrest und die anderen Reste jeweils für ein Wasserstoffatom stehen, und M⁺ für ein Alkalimetallkation oder ein Ammoniumion steht,
b) 2,5 bis 8,5 Gew.-% mindestens eines amphoteren und/oder zwitterionischen Tensids einer der zuvor genannten allgemeinen Formeln (iii), (v), (vi) oder (vii),
c) 0,75 bis 8 Gew.-% mindestens eines nichtionisches Tensids und/oder nichtionischen Emulgators einer der zuvor genannten allgemeinen Formeln (II), (III) oder RO-[G]ₓ und
d) mindestens einen konditionierenden Wirkstoff, ausgewählt aus der Gruppe der Proteinhydrolysate, der kationischen Polymere, der Vitamine, der pflanzlichen Öle und Glycerin.

Innerhalb dieser Ausführungsform ist es mehr bevorzugt, wenn die erfindungsgemäßen Reinigungsmittel - bezogen auf ihr Gesamtgewicht -
a) 1,2 bis 4 Gew.-% mindestens einer der unter den INCI-Bezeichnungen Natrium Cocoyl Methyl Isethionate, Kalium Cocoyl Methyl Isethionate, Ammonium Cocoyl Methyl Isethionate, Natrium Lauroyl Methyl Isethionate, Kalium Lauroyl Methyl Isethionate, Ammonium Lauroyl Methyl Isethionate, Natrium Myristoyl Methyl Isethionate, Kalium Myristoyl Methyl Isethionate und Ammonium Myristoyl Methyl Isethionate bekannten Verbindungen,
b) 3 bis 7,5 Gew.-% mindestens eines der unter den INCI-Bezeichnungen Cocamidopropylbetain und/oder Cocoamphodiacetate bekannten amphoteren und/oder zwitterionischen Tenside,
c) 1 bis 6 Gew.-% mindestens eines Alkyl(oligo)glycosids der allgemeinen Formel RO-[G]ₓ, in der R für einen Alkyl- und/oder Alkenylrest mit 4 bis 22 C-Atomen, G für einen Zuckerrest mit 5 oder 6 C-Atomen und x für Zahlen von 1 bis 10 steht, und
d) mindestens ein kationisches Polymer, ausgewählt aus kationischen Cellulosederivaten, hydrophob modifizierten kationischen Cellulosederivaten, kationischen Guar-Derivaten und/oder Glycerin enthalten.

Insbesondere bevorzugte milde kosmetische Reinigungsmittel dieser Ausführungsform enthalten - bezogen auf ihr Gesamtgewicht -
d) mindestens eines der unter der INCI-Bezeichnungen Guar Hydroxypropyltrimonium Chloride, Polyquaternium-10 und/oder Polyquaternium-67bekannten kationischen Polymere und Glycerin.

Wie bereits ausgeführt, eignen sich die erfindungsgemäßen milden Reinigungsmittel als Haut- und/oder Haarreinigungsmittel für Babys und Kleinkinder.
Für solche Anwendungsformen ist es von Vorteil, wenn die Reinigungsmittel zusätzlich einen Bitterstoff enthalten, der eine Molmasse von mindestens 250 g/mol aufweist, und der in dem Reinigungsmittel bei 20°C zu mindestens 10 mg/l löslich ist.

Beispiele für geeignete Bitterstoffe sind quartäre Ammoniumverbindungen, die sowohl im Kation als auch im Anion eine aromatische Gruppe enthalten. Solche Verbindungen sind kommerziell erhältlich, z.B. unter den Warenzeichen Bitrex^{®} und Indigestin^{®} (Benzyldiethyl((2,6-Xylylcarbamoyl)methyl)ammoniumbenzoat) oder unter der Bezeichnung Denatonium Benzoate.

Der oder die Bitterstoff(e) können in den erfindungsgemäßen Zusammensetzungen bevorzugt in Mengen von 0,0005 bis 0,1 Gew.-%, bezogen auf die gesamte Zubereitung, enthalten sein. Besonders bevorzugt sind Mengen von 0,001 bis 0,05 Gew.-%.

Unter "ethoxylierten Wirkstoffen" werden bevorzugt Wirkstoffe verstanden, die die folgenden Gruppierungen umfassen:
1) H-(O-CH₂CH₂)n-OH,
   worin n für ganze Zahlen zwischen 1 und 100.000 steht,
2) H-(O-CH(CH₃)CH₂)ₙ-OH,
   worin n für ganze Zahlen zwischen 1 und 100.000 steht,
3) R-(O-CH₂CH₂)ₙ-OH,
   worin R für einen Alkyl- oder Alkenylrest mit 2 bis 30 C-Atomen und n für Zahlen zwischen 1 und 10.000 steht,

Unter "ethoxylierten Wirkstoffen" werden bevorzugt Wirkstoffe verstanden, die die folgenden Gruppierungen umfassen:
1) H-(O-CH₂CH₂)ₙ-OH,
   worin n für ganze Zahlen zwischen 1 und 100.000 steht,
2) H-(O-CH(CH₃)CH₂)ₙ-OH,
   worin n für ganze Zahlen zwischen 1 und 100.000 steht,
3) R-(O-CH₂CH₂)ₙ-OH,
   worin R für einen Alkyl- oder Alkenylrest mit 2 bis 30 C-Atomen und n für Zahlen zwischen 1 und 10.000 steht,
4) R-(O-CH₂CH₂)ₙ-OSO₃H,
   worin R für einen Alkyl- oder Alkenylrest mit 2 bis 30 C-Atomen und n für Zahlen zwischen 1 und 10.000 steht, und
5) -(O-CH₂CH₂)ₙ-,
   worin n für ganze Zahlen zwischen 2 und 100.000 steht.

Die erfindungsgemäßen Mittel sind bevorzugt frei von Polyethylenglycolen der allgemeinen Formel 1), d.h. sie enthalten bevorzugt weder Ethylenglycol (n = 1) noch Produkte mit einem Polymeristaionsgrad Pₙ= 2-4 (Diethylenglycol, Triethylenglycol und Tetraethylenglycol) noch Polyethylenglycole mit höheren Polymerisationsgraden Pₙ von ca. 5 bis 100.000, die nicht mehr mol.-einheitlich herstellbar, sondern polydispers sind.
Sie sind bevorzugt auch frei von Polypropylenglycolen der allgemeinen Formel 2), d.h. sie enthalten weder Propylenglycol (n = 1) noch Produkte mit einem Polymeristaionsgrad Pₙ= 2-4 (Dipropylenglycol, Tripropylenglycol und Tetrapropylenglycol) noch Polypropylenglycole mit höheren Polymerisationsgraden Pₙ von ca. 5 bis 100.000, die nicht mehr mol.-einheitlich herstellbar, sondern polydispers sind.

Es hat sich gezeigt, dass vorzugsweise auch auf den Einsatz sonstiger ethoxylierter Verbindungen verzichtet werden sollte. Insbesondere nichtionische Tenside vom Typ der Alkyl- oder Alkenylethoxylate vermindern ebenfalls die positiven Effekte der erfindungsgemäßen Mittel und sollten deshalb in den erfindungsgemäßen Reinigungsmitteln nicht eingesetzt werden. Deshalb enthalten erfindungsgemäß bevorzugte Reinigungsmittel auch keine Verbindungen der Formel 3).

Bevorzugt sind weiterhin erfindungsgemäße Mittel, die im Wesentlichen frei sind von Alkylethersulfaten der zuvor genannten Formel 4), wobei unter "im Wesentlichen frei" die zuvor genannte Definition zu verstehen ist.

In besonders bevorzugten erfindungsgemäßen Mitteln wird gänzlich auf den Einsatz von Verbindungen verzichtet, die ethoxylierte Gruppierungen aufweisen.
Bevorzugte erfindungsgemäße Reinigungsmittel enthalten daher keine Verbindung, welche die Gruppierung -(O-CH₂CH₂)ₙ-O- mit n = 1 - 10.000 enthält. Besonders bevorzugte erfindungsgemäße Haut- und/oder Haarreinigungsmittel sind dadurch gekennzeichnet, dass sie keine Verbindungen der Formel 5) aufweisen.

Empfindliche Kopfhaut kann durch Schuppenbefall und den damit verbundenen Juckreiz entstehen. Sie muss mit besonders milden Reinigungszusammensetzungen behandelt werden, die Kopfschuppen wirksam und nachhaltig entfernen und dabei die Kopfhaut pflegen, ohne sie zusätzlich zu belasten.
Es wurde festgestellt, dass sich erfindungsgemäße Reinigungsmittel auch für eine Anwendung als Antischuppen-Reinigungszubereitung eignen.
Für die Konfektionierungsform als Antischuppenmittel enthalten erfindungsgemäße Mittel - bezogen auf ihr Gesamtgewicht - bevorzugt 0,01 bis 10 Gew.-%, mehr bevorzugt 0,025 bis 7,5 Gew.-%, besonders bevorzugt 0,05 bis 5 Gew.-% und insbesondere 0,075 bis 3 Gew.-% mindestens eines Antischuppenwirkstoffs.
Geeignete Antischuppenwirkstoffe können ausgewählt sein aus Piroctone Olamine, Climbazol, Zink Pyrithion, Ketoconazole, Salicylsäure, Schwefel, Selensulfid, Teerpräparaten, Undecensäurederivaten, Klettenwurzelextrakten, Pappelextrakten, Brennesselextrakten, Walnussschalenextrakten, Birkenextrakten, Weidenrindenextrakten, Rosmarinextrakten und/oder Arnikaextrakten.
Bevorzugt sind Climbazol, Zink Pyrithion und Piroctone Olamine.

Weitere Wirk-, Hilfs- und Zusatzstoffe, die erfindungsgemäße Reinigungsmitteln enthalten können, sind beispielsweise:
- Pflanzenextrakte,
- Feuchthaltemittel,
- Parfums,
- UV-Filter,
- Verdickungsmittel wie Gelatine oder Pflanzengumme, beispielsweise Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone und Schichtsilikate wie z. B. Bentonit oder vollsynthetische Hydrokolloide wie z. B. Polyvinylalkohol, die Ca-, Mg- oder Zn - Seifen,
- Strukturanten wie Maleinsäure und Milchsäure,
- Dimethylisosorbid,
- Cyclodextrine,
- faserstrukturverbessernde Wirkstoffe, insbesondere Mono-, Di- und Oligosaccharide wie beispielsweise Glucose, Galactose, Fructose, Fruchtzucker und Lactose,
- Farbstoffe zum Anfärben des Mittels,
- Substanzen zur Einstellung des pH-Wertes, beispielsweise α- und β-Hydroxycarbonsäuren wie Citronensäure, Milchsäure, Äpfelsäure, Glycolsäure,
- Wirkstoffe wie Bisabolol,
- Komplexbildner wie EDTA, NTA, β-Alanindiessigsäure und Phosphonsäuren,
- Ceramide. Unter Ceramiden werden N-Acylsphingosin (Fettsäureamide des Sphingosins) oder synthetische Analogen solcher Lipide (sogenannte Pseudo-Ceramide) verstanden,
- Treibmittel wie Propan-Butan-Gemische, N₂O, Dimethylether, CO₂ und Luft,
- Antioxidantien,
- Konservierungsmittel, wie beispielsweise Natriumbenzoat oder Salicylsäure,
- Viskositätsregler wie Salze (NaCl).

Die erfindungsgemäßen milden Reinigungsmittel weisen bevorzugt einen pH-Wert im Bereich von 3 bis 6, mehr bevorzugt von 3,5 bis 6, besonders bevorzugt von 4 bis 5,5 und insbesondere von 4,5 bis 5,5 auf.

Die erfindungsgemäßen Reinigungsmittel weisen den Vorteil auf, dass sie besonders mild und gut hautverträglich sind.
Sie weisen weiterhin eine hervorragende Pflegeleistung - insbesondere bei der Anwendung der Reinigungsmittel auf den Haaren - auf. Eine Zugabe von Silikonen ist nicht erforderlich.
Alle formulierungstechnisch notwendigen Rohstoffe können in dem kosmetischen Träger - vorzugsweise in Wasser - gelöst werden, so dass die Zugabe ethoxylierter Lösungsvermittler ebenfalls nicht erforderlich ist.
Die erfindungsgemäßen Reinigungsmittel sind äußerst schaumstark und liefern einen dichten Schaum, der sich leicht auf der Anwendungsoberfläche verteilen lässt. Auf den Einsatz von Sulfattensiden kann demnach verzichtet werden.
Die erfindungsgemäßen Reinigungsmittel weisen eine ausgezeichnete Textur auf und sind fließfähig.
Weiterhin vorteilhaft ist, dass die erfindungsgemäßen Reinigungsmittel auch bei niedrigeren pH-Werten stabil sind. Eine gut verträgliche Säurekonservierung der Mittel ist demnach möglich.

Ein zweiter Gegenstand der Erfindung ist die Verwendung der erfindungsgemäßen milden kosmetischen Reinigungsmittel als sensitive Gesichtsreinigungsmittel, als Baby-Shampoo und/oder als Baby-Badezubereitung.

### Beispiele:

### 1) Formulierungsbeispiele

Es wurden die folgenden erfindungsgemäßen Reinigungsmittel hergestellt (die Mengenangaben beziehen sich - sofern nicht anders angegeben - auf Gew.-%):

**Tabelle 1**

| | **Baby-Shampoo 1** | **Kids Shampoo 2** |
|---|---|---|
| **Iselux^{®1} LQ-CLR-SB** | 5 | 5 |
| **Coco-Glucoside (50%AS)** | 7 | 4 |
| **Cocamidopropylbetain (40%AS)** | 13 | 13 |
| **Polyquaternium-10** | 0,1 | |
| **Polyquaternium-67** | | 0,3 |
| **Glycerin** | 5 | 5 |
| **Panthenol** | 0,2 | |
| **Bitrex^{®2}** | | 0,08 |
| **Säuerungsmittel, Konservierungsmittel** | q.s. | q.s. |
| **Wasser** | ad 100 | ad 100 |

In den Reinigungsmitteln der Tabelle 1 wurden die folgenden Handelsprodukte eingesetzt:
1 INCI-Bezeichnung: Sodium Lauroyl Methyl Isethionate; 32%AS; Innospec
2 INCI-Bezeichnung: Wasser, Denatonium Benzoate; 2,5%AS; Macfarlan Smith Ltd.

Die Reinigungsmittel 1 und 2 sind besonders mild, pflegend und nur sehr geringfügig hautirritierend.

### 2) Beurteilung der Formulierungsbeispiele

Die Reinigungsmittel 1 und 2 wurden in einem Test (Reaktionszeitmethode) mit verschiedenen im Handel erhältlicher Baby- und Kinder-Shampoos verglichen. Dabei wurden die Shampoos jeweils zu jeweils 50% mit Wasser verdünnt.
Als Referenz diente eine 5%ige Natriumlaurethsulfatlösung.
Ein Wert Q > 1,20 steht für reizend; ein Wert Q < 0,8 steht für geringfügig reizend.
Die Ergebnisse des Tests sind der nachfolgenden Tabelle 2 zu entnehmen:

**Tabelle 2**

| | **Konzentration (in Wasser)** | **pH-Wert** | **Reaktionszeit methode [Q]** | **Evaluierung** |
|---|---|---|---|---|
| **Baby-Shampoo 1** | 50% | 4,8 | 0,36 | geringfügig reizend |
| **Kids Shampoo 2** | 50% | 4,9 | 0,37 | geringfügig reizend |
| **Bübchen Baby Shampoo** | 50% | 7,3 | 0,42 | geringfügig reizend |
| **Bübchen Kinder Shampoo** | 50% | 5,7 | 0,59 | geringfügig reizend |
| **Bübchen Kids Shampoo** | 50% | 5,7 | 1,23 | reizend |
| **Penaten Baby Bad & Shampoo** | 50% | 5,8 | 0,43 | geringfügig reizend |
| **Referenz (SLES, 5% in Wasser)** | 5% | 7,2 | 1,00 | moderat reizend |

## Patentansprüche

1. Mildes kosmetisches Reinigungsmittel, enthaltend in einem geeigneten Träger
a) 1 bis 6 Gew.-% mindestens eines anionischen Tensids der nachfolgenden Formel (I), 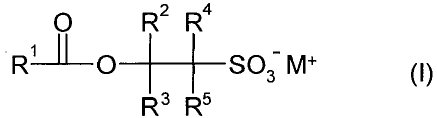 in der
- R¹ für einen linearen oder verzweigten, gesättigten oder ungesättigten Alkylrest mit 6 bis 30 Kohlenstoffatomen steht,
- mindestens einer der Reste R² bis R⁵ für einen C₁-C₄-Alkylrest, und die anderen Reste unabhängig voneinander für ein Wasserstoffatom oder einen C₁-C₄-Alkylrest stehen, und
- M⁺ für ein Ammonium-, ein Alkanolammonium oder ein Metallkation steht,
b) 2 bis 10 Gew.-% mindestens eines amphoteren und/oder zwitterionischen Tensids und
c) 0,5 bis 10 Gew.-% mindestens eines nichtionischen Tensids und/oder mindestens eines nichtionischen Emulgators, ausgewählt aus mindestens einer der nachfolgenden Gruppen der
- Alkyl(oligo)glycoside,
- Aminoxide und/oder
- Glycerinmono- und/oder -di-(C₆-C₂₄-)Carbonsäureester,
wobei sich die Mengenangaben auf das Gesamtgewicht des Reinigungsmittels beziehen, und wobei es weniger als 0,5 Gew.-%, mehr bevorzugt weniger als 0,25 Gew.-% und besonders bevorzugt weniger als 0,1 Gew.-% ethoxylierte Wirkstoffe enthält.

2. Mildes kosmetisches Reinigungsmittel nach Anspruch 1, **dadurch gekennzeichnet, dass** es
die Tenside/Emulgatoren a), b) und c) in einem Gewichtsverhältnis von (1-2) : (2-5) : (1-3) enthält.

3. Mildes kosmetisches Reinigungsmittel nach einem der Ansprüche 1 oder 2, **dadurch gekennzeichnet, dass** es ein anionisches Tensid gemäß Formel (I) enthält, in der
- R¹ für einen linearen oder verzweigten, gesättigten oder ungesättigten Alkylrest mit 8 bis 18 Kohlenstoffatomen steht und
- mindestens einer der Reste R² bis R⁵ für einen Methylrest, und die anderen Reste für ein Wasserstoffatom stehen.

4. Mildes kosmetisches Reinigungsmittel nach einem der Ansprüche 1 bis 3, **dadurch gekennzeichnet, dass** es ein anionisches Tensid gemäß Formel (I) enthält, in der
- R¹ für einen C₈-, C₁₀-, C₁₂-, C₁₄-, C₁₆-Rest oder für Gemische dieser Fettsäurereste steht, wie sie erhalten werden, wenn sich die Fettsäure(n) aus natürlichen Ölen wie beispielsweise Kokosöl ableitet(n), und
- einer der Reste R² bis R⁵ für einen Methylrest, und die anderen Reste für ein Wasserstoffatom stehen.

5. Mildes kosmetisches Reinigungsmittel nach einem der Ansprüche 1 bis 4, **dadurch gekennzeichnet, dass** es mindestens eine Verbindung der nachfolgenden Formeln (i) bis (vii) enthält, in denen der Rest R für einen geradkettigen oder verzweigten, gesättigten oder ein- oder mehrfach ungesättigten Alkyl- oder Alkenylrest mit 7 bis 23 Kohlenstoffatomen (Formeln (i) und (ii)) oder für einen geradkettigen oder verzweigten, gesättigten oder ein- oder mehrfach ungesättigten Alkyl- oder Alkenylrest mit 8 bis 24 Kohlenstoffatomen (Formeln (iii) bis (vii)) steht:

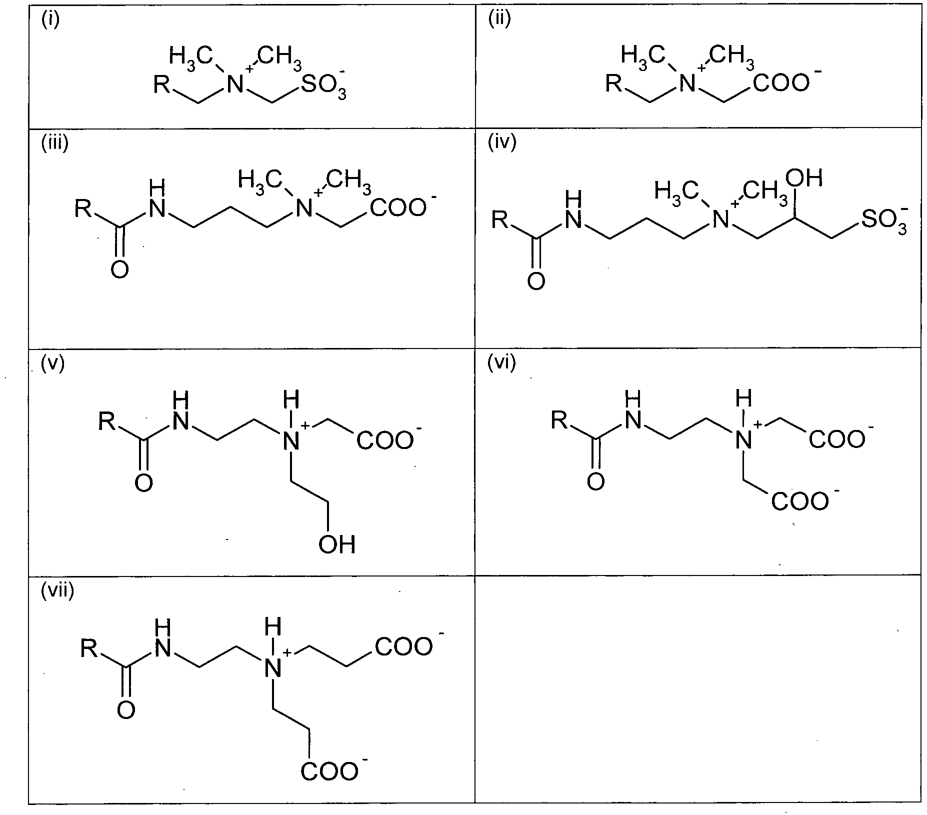

6. Mildes kosmetisches Reinigungsmittel nach Anspruch 5, **dadurch gekennzeichnet, dass** es amphotere und/oder zwitterionische Tenside der Formeln (iii), (v), (vi) und (vii) enthält, in denen sich der Rest R von Kokosfett ableitet.

7. Mildes kosmetisches Reinigungsmittel nach einem der Ansprüche 1 bis 6, **dadurch gekennzeichnet, dass** es als nichtionisches Tensid und/oder nichtionischen Emulgator mindestens ein Alkyl(oligo)glycosid der allgemeinen Formel RO-[G]ₓ enthält, in der R für einen Alkyl- und/oder Alkenylrest mit 4 bis 22 C-Atomen, G für einen Zuckerrest mit 5 oder 6 C-Atomen und x für Zahlen von 1 bis 10 steht und/oder mindestens ein Aminoxid einer der nachfolgenden Formeln (II) oder (III), 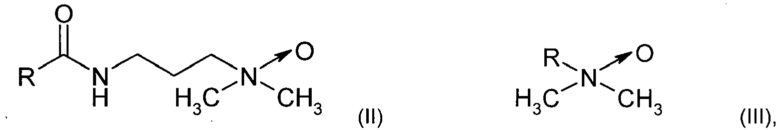 in denen R jeweils für einen geradkettigen oder verzweigten, gesättigten oder ein- oder mehrfach ungesättigten Alkyl- oder Alkenylrest mit 6 bis 24 Kohlenstoffatomen, bevorzugt mit 8 bis 18 Kohlenstoffatomen steht.

8. Mildes kosmetisches Reinigungsmittel nach einem der Ansprüche 1 bis 7, **dadurch gekennzeichnet, dass** es - bezogen auf sein Gesamtgewicht -
a) 1,1 bis 5 Gew.-% mindestens eines anionischen Tensids der zuvor genannten Formel (I), in der der Rest R¹ für einen linearen oder verzweigten, gesättigten oder ungesättigten Alkylrest mit 8 bis 18 Kohlenstoffatomen steht, mindestens einer der Reste R² bis R⁵ für einen Methylrest und die anderen Reste jeweils für ein Wasserstoffatom stehen, und M⁺ für ein Alkalimetallkation oder ein Ammoniumion steht,
b) 2,5 bis 8,5 Gew.-% mindestens eines amphoteren und/oder zwitterionischen Tensids einer der zuvor genannten allgemeinen Formeln (iii), (v), (vi) oder (vii), und
c) 0,75 bis 8 Gew.-% mindestens eines nichtionisches Tensids und/oder nichtionischen Emulgators einer der zuvor genannten allgemeinen Formeln (II), (III) oder RO-[G]ₓ enthält.

9. Mildes kosmetisches Reinigungsmittel nach einem der Ansprüche 1 bis 8, **dadurch gekennzeichnet, dass** es zusätzlich mindestens einen konditionierenden Wirkstoff enthält, der ausgewählt ist aus mindestens einem Wirkstoff der folgenden Gruppe der
- Proteinhydrolysate,
- kationischen Polymere,
- Vitamine,
- pflanzlichen Öle,
- Glycerin.

10. Mildes kosmetisches Reinigungsmittel nach Anspruch 9, **dadurch gekennzeichnet, dass** es mindestens ein kationisches Polymer, ausgewählt aus kationischen Cellulosederivaten, hydrophob modifizierten kationischen Cellulosederivaten und/oder kationischen Guar-Derivaten, und Glycerin enthält.

11. Mildes kosmetisches Reinigungsmittel nach einem der Ansprüche 1 bis 10, **dadurch gekennzeichnet, dass** es zusätzlich mindestens einen Bitterstoff enthält, der eine Molmasse von mindestens 250 g/mol aufweist und in dem Reinigungsmittel bei 20 °C zu mindestens 10 mg/l löslich ist.

12. Mildes kosemtisches Reinigungsmittel nach einem der Ansprüche 1 bis 11, **dadurch gekennzeichnet, dass** es - bezogen auf sein Gesamtgewicht -
a) 1,1 bis 5 Gew.-% mindestens eines anionischen Tensids der zuvor genannten Formel (I), in der der Rest R¹ für einen linearen oder verzweigten, gesättigten oder ungesättigten Alkylrest mit 8 bis 18 Kohlenstoffatomen steht, mindestens einer der Reste R² bis R⁵ für einen Methylrest und die anderen Reste jeweils für ein Wasserstoffatom stehen, und M⁺ für ein Alkalimetallkation oder ein Ammoniumion steht,
b) 2,5 bis 8,5 Gew.-% mindestens eines amphoteren und/oder zwitterionischen Tensids einer der zuvor genannten allgemeinen Formeln (iii), (v), (vi) oder (vii),
c) 0,75 bis 8 Gew.-% mindestens eines nichtionisches Tensids und/oder nichtionischen Emulgators einer der zuvor genannten allgemeinen Formeln (II), (III) oder RO-[G]ₓ,
d) mindestens einen konditionierenden Wirkstoff, ausgewählt aus der Gruppe der Proteinhydrolysate, der kationischen Polymere, der Vitamine, der pflanzlichen Öle und Glycerin, und
e) mindestens einen Bitterstoff enthält, der eine Molmasse von mindestens 250 g/mol aufweist, und in dem Reinigungsmittel bei 20 °C zu mindestens 10 mg/l löslich ist.

13. Mildes kosmetisches Reinigungsmittel nach einem der Ansprüche 1 bis 12, **dadurch gekennzeichnet, dass** es einen pH-Wert im Bereich von 4 bis 5,5, bevorzugt von 4,5 bis 5,5 aufweist.

14. Verwendung eines milden kosmetischen Reinigungsmittels nach einem der Ansprüche 1 bis 13 als sensitives Gesichtsreinigungsmittel, als Baby-Shampoo und/oder als Baby-Badezubereitung.

## Claims

1. A mild cosmetic cleansing agent containing, in a suitable carrier,
a) 1 to 6 wt.% of at least one anionic surfactant of the following formula (I), 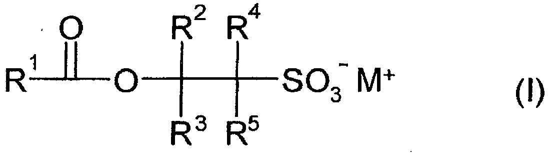 in which
- R¹ represents a linear or branched, saturated or unsaturated alkyl group having 6 to 30 carbon atoms,
- at least one of the groups R² to R⁵ represents a C₁-C₄ alkyl group, and the other groups represent, independently of one another, a hydrogen atom or a C₁-C₄ alkyl group, and
- M⁺ represents an ammonium cation, an alkanol ammonium cation or a metal cation,
b) 2 to 10 wt.% of at least one amphoteric and/or zwitterionic surfactant, and
c) 0.5 to 10 wt.% of at least one non-ionic surfactant and/or at least one non-ionic emulsifier, selected from at least one of the following groups consisting of
- alkyl (oligo)glycosides,
- amine oxides, and/or
- glycerol monO(C₆-C₂₄)carboxylic acid esters and/or glycerol di(C₆-C₂₄)carboxylic acid esters,
the specified amounts referring to the total weight of the cleansing agent, wherein said agent contains less than 0.5 wt.%, more preferably less than 0.25 wt.%, and particularly preferably less than 0.1 wt.%, ethoxylated active ingredients.

2. The mild cosmetic cleansing agent according to claim 1, **characterized in that** it contains the surfactants/emulsifiers a), b) and c) in a weight ratio of (1-2):(2-5):(1-3).

3. The mild cosmetic cleansing agent according to claim 1 or 2, **characterized in that** it contains an anionic surfactant according to formula (I), in which
- R¹ represents a linear or branched, saturated or unsaturated alkyl group having 8 to 18 carbon atoms, and
- at least one of the groups R² to R⁵ represents a methyl group, and the other groups represent a hydrogen atom.

4. The mild cosmetic cleansing agent according to one of claims 1 to 3, **characterized in that** it contains an anionic surfactant according to formula (I), in which
- R¹ represents a C₈, C₁₀, C₁₂, C₁₄ or C₁₆ functional group or mixtures of these fatty acid groups as are obtained when the fatty acid(s) is/are derived from natural oils such as coconut oil, and
- one of the groups R² to R⁵ represents a methyl group, and the other groups represent a hydrogen atom.

5. The mild cosmetic cleansing agent according to one of claims 1 to 4, **characterized in that** it contains at least one compound of the following formulae (i) to (vii), in which the group R represents a straight-chain or branched, saturated or monounsaturated or polyunsaturated alkyl or alkenyl group having 7 to 23 carbon atoms (formulae (i) and (ii)), or a straight-chain or branched, saturated or monounsaturated or polyunsaturated alkyl or alkenyl group having 8 to 24 carbon atoms (formulae (iii) to (vii)):

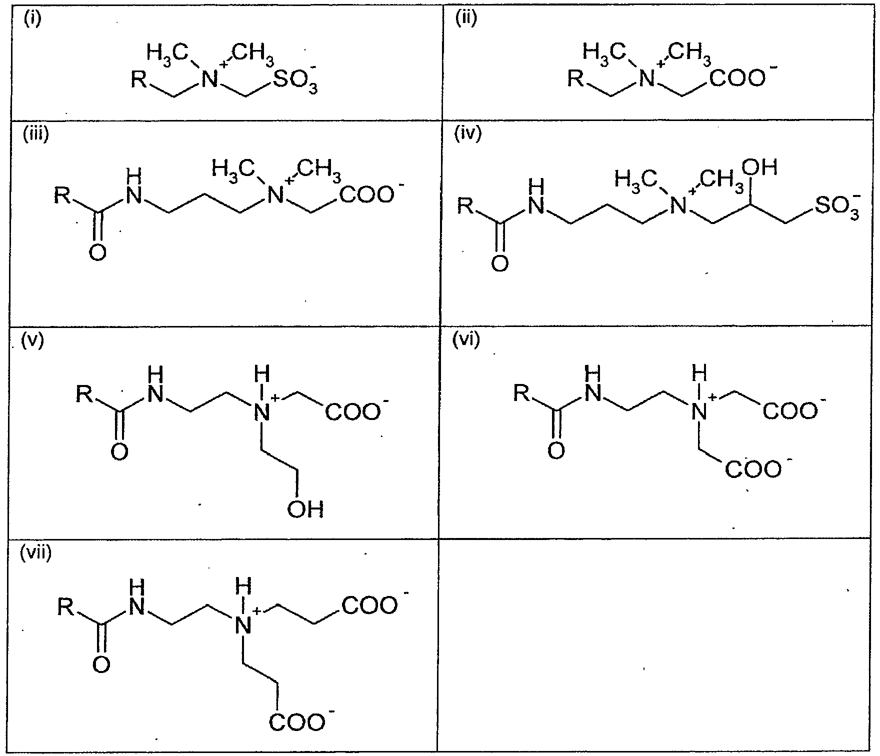

6. The mild cosmetic cleansing agent according to claim 5, **characterized in that** it contains amphoteric and/or zwitterionic surfactants of formulae (iii), (v), (vi) and (vii), in which the group R is derived from coconut fat.

7. The mild cosmetic cleansing agent according to one of claims 1 to 6, **characterized in that** it contains, as the non-ionic surfactant and/or non-ionic emulsifier, at least one alkyl (oligo)glycoside of the general formula RO-[G]ₓ, in which R represents an alkyl and/or alkenyl group having 4 to 22 carbon atoms, G represents a sugar residue having 5 or 6 carbon atoms, and x represents numbers from 1 to 10, and/or at least one amine oxide of one of the following formulae (II) or (III), 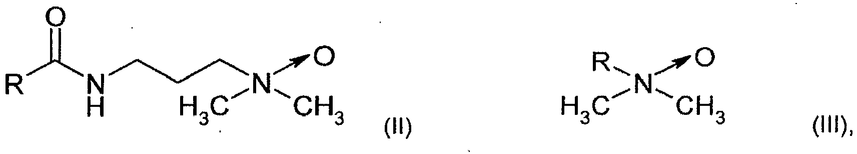 in which R in each case represents a straight-chain or branched, saturated or monounsaturated or polyunsaturated alkyl or alkenyl group having 6 to 24 carbon atoms, preferably having 8 to 18 carbon atoms.

8. The mild cosmetic cleansing agent according to one of claims 1 to 7, **characterized in that** it contains, based on its total weight,
a) 1.1 to 5 wt.% of at least one anionic surfactant of the aforementioned formula (I), in which the group R¹ represents a linear or branched, saturated or unsaturated alkyl group having 8 to 18 carbon atoms, at least one of the groups R² to R⁵ represents a methyl group, and the other groups each represent a hydrogen atom, and M⁺ represents an alkali metal cation or an ammonium ion,
b) 2.5 to 8.5 wt.% of at least one amphoteric and/or zwitterionic surfactant of one of the aforementioned general formulae (iii), (v), (vi) or (vii), and
c) 0.75 to 8 wt.% of at least one non-ionic surfactant and/or non-ionic emulsifier of one of the aforementioned general formulae (II), (III) or RO-[G]ₓ.

9. The mild cosmetic cleansing agent according to one of claims 1 to 8, **characterized in that** it additionally contains at least one conditioning active ingredient selected from at least one active ingredient of the following group consisting of
- protein hydrolyzates,
- cationic polymers,
- vitamins,
- vegetable oils,
- glycerol.

10. The mild cosmetic cleansing agent according to claim 9, **characterized in that** it contains at least one cationic polymer selected from cationic cellulose derivatives, hydrophobically modified cationic cellulose derivatives and/or cationic guar derivatives, and glycerol.

11. The mild cosmetic cleansing agent according to one of claims 1 to 10, **characterized in that** it additionally contains at least one bittern that has a molar mass of at least 250 g/mol and in which cleansing agent is soluble at 20°C in a proportion of at least 10 mg/l.

12. The mild cosmetic cleansing agent according to one of claims 1 to 11, **characterized in that** it contains, based on its total weight,
a) 1.1 to 5 wt.% of at least one anionic surfactant of the aforementioned formula (I), in which the group R¹ represents a linear or branched, saturated or unsaturated alkyl group having 8 to 18 carbon atoms, at least one of the groups R² to R⁵ represents a methyl group, and the other groups each represent a hydrogen atom, and M⁺ represents an alkali metal cation or an ammonium ion,
b) 2.5 to 8.5 wt.% of at least one amphoteric and/or zwitterionic surfactant of one of the aforementioned general formulae (iii), (v), (vi) or (vii),
c) 0.75 to 8 wt.% of at least one non-ionic surfactant and/or non-ionic emulsifier of one of the aforementioned general formulae (II), (III) or RO-[G]ₓ,
d) at least one conditioning active ingredient selected from the group consisting of protein hydrolyzates, cationic polymers, vitamins, vegetable oils and glycerol, and
e) at least one bittern that has a molar mass of at least 250 g/mol and in which cleansing agent is soluble at 20°C in a proportion of at least 10 mg/l.

13. The mild cosmetic cleansing agent according to one of claims 1 to 12, **characterized in that** it has a pH in the range of from 4 to 5.5, preferably from 4.5 to 5.5.

14. The use of a mild cosmetic cleansing agent according to one of claims 1 to 13 as a sensitive face cleansing agent, as a baby shampoo and/or as a baby bath preparation.

## Revendications

1. Agent nettoyant cosmétique doux contenant dans un support approprié
a) de 1 à 6 % en poids d'au moins un tensioactif anionique de la formule (I) suivante, 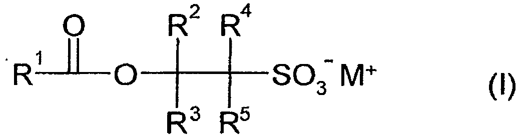 où
- R¹ représente un résidu alkyle linéaire ou ramifié, saturé ou insaturé ayant de 6 à 30 atomes de carbone,
- au moins un des résidus R² à R⁵ représente un résidu alkyle en C₁-C₄, et les autres résidus représentent, indépendamment les uns des autres, un atome d'hydrogène ou d'un résidu alkyle en C₁-C₄, et
- M⁺ représente un cation d'ammonium, d'ammonium alcalin ou de métal,
b) de 2 à 10 % en poids d'au moins un tensioactif amphotère et/ou zwitterionique et
c) de 0,5 à 10 % en poids d'au moins un tensioactif non ionique et/ou au moins un émulsifiant non ionique, choisi parmi au moins l'un des groupes suivants
- alkyl(oligo)glycosides,
- amine-oxyde et/ou,
- ester d'acide mono et/ou dicarboxylique (C₆-C₂₄) de glycérine,
où les quantités indiquées sont basées sur le poids total de l'agent nettoyant, et où il contient moins de 0,5 % en poids, plus préférablement moins de 0,25 % en poids et particulièrement préférablement moins de 0,1 % en poids de principes actifs éthoxylés.

2. Agent nettoyant cosmétique doux selon la revendication 1, **caractérisé en ce qu'**il contient les tensioactifs/émulsifiants a), b) et c) dans un rapport de poids de (1-2) : (2-5) : (1-3).

3. Agent nettoyant cosmétique doux selon l'une quelconque des revendications 1 ou 2, **caractérisé en ce qu'**il contient un tensioactif anionique selon la formule (I), dans lequel :
- R¹ représente un résidu alkyle linéaire ou ramifié, saturé ou insaturé ayant 8 à 18 atomes de carbone, et
- au moins un des résidus R² à R⁵ représente un résidu méthyle, et les autres résidus représentent un atome d'hydrogène.

4. Agent nettoyant cosmétique doux selon l'une quelconque des revendications 1 à 3, **caractérisé en ce qu'**il contient un tensioactif anionique selon la formule (I), dans lequel
- R¹ représente un résidu en C₈, C₁₀, C₁₂, C₁₄ ou C₁₆ ou des mélanges de ces résidus d'acides gras, tels qu'ils sont obtenus lorsque l'(les) acide(s) gras est (sont) dérivé(s) d'huiles naturelles telles que l'huile de noix de coco, et
- un des résidus R² à R⁵ représente un résidu méthyle et les autres résidus représentent un atome d'hydrogène.

5. Agent nettoyant cosmétique doux selon l'une quelconque des revendications 1 à 4, **caractérisé en ce qu'**il contient au moins un composé des formules suivantes (i) à (vii), dans lesquelles le résidu R représente un résidu alkyle ou alcényle, linéaire ou ramifié, saturé ou mono- ou polyinsaturés avec 7 à 23 atomes de carbone (formules (i) et (ii)) ou un résidu alkyle ou alcényle linéaire ou ramifié, saturé ou mono- ou polyinsaturés avec 8 à 24 atomes de carbone (formules (iii) à (vii)) :

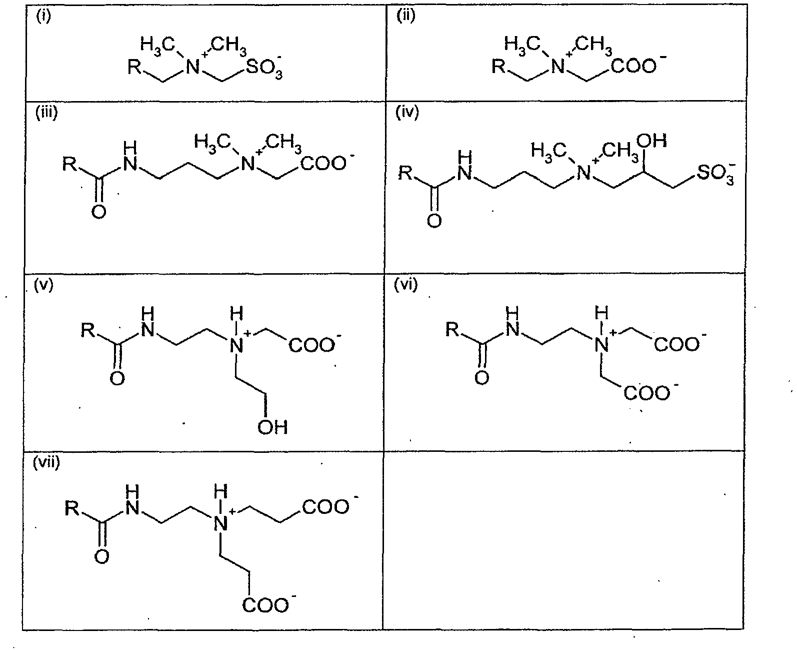

6. Agent nettoyant cosmétique doux selon la revendication 5, **caractérisé en ce qu'**il contient des tensioactifs amphotères et/ou zwittérioniques des formules (iii), (v), (vi) et (vii), dans lesquelles le résidu R est dérivé de graisse de coco.

7. Agent nettoyant cosmétique doux selon l'une quelconque des revendications 1 à 6, **caractérisé en ce qu'**il contient, comme tensioactif non ionique et/ou comme émulsifiant non ionique, au moins un alkyle(oligo)glycoside de la formule générale RO-[G]ₓ, dans laquelle R représente un résidu alkyle ou alcényle avec 4 à 22 atomes de carbone, G représente un résidu de sucre ayant 5 ou 6 atomes de carbone et x représente des nombres de 1 à 10 et/ou au moins un amine-oxyde de l'une des formules suivantes (II) ou (III), 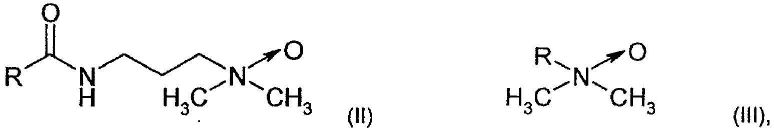 dans laquelle R représente respectivement un résidu alkyle ou alcényle linéaire ou ramifié, saturé ou mono- ou polyinsaturé ayant 6 à 24 atomes de carbone, de préférence ayant 8 à 18 atomes de carbone.

8. Agent nettoyant cosmétique doux selon l'une quelconque des revendications 1 à 7, **caractérisé en ce qu'**il contient - rapporté à son poids total,
a) de 1,1 à 5 % en poids d'au moins un tensioactif anionique de la formule ci-dessus (I), dans laquelle le résidu R¹ représente un résidu alkyle linéaire ou ramifié, saturé ou insaturé avec 8 à 18 atomes de carbone, au moins un des résidus R² à R⁵ représente un résidu méthyle et les autres résidus représentent respectivement un atome d'hydrogène, et M+ représente un cation de métal alcalin ou un ion ammonium,
b) de 2,5 à 8,5 % en poids d'au moins un tensioactif amphotère et/ou zwittérionique d'une des formules générales mentionnées ci-dessus (iii), (v), (vi) ou (vii), et
c) de 0,75 à 8 % en poids d'au moins un tensioactif non ionique et/ou un émulsifiant non ionique d'une des formules générales mentionnées ci-dessus (II), (III) ou RO-[G]ₓ.

9. Agent nettoyant cosmétique doux selon l'une quelconque des revendications 1 à 8, **caractérisé en ce qu'**il contient en plus au moins un principe actif de conditionnement sélectionné parmi au moins un principe actif du groupe suivant des
- hydrolysats de protéines,
- polymères cationiques,
- vitamines,
- huiles végétales,
- glycérine.

10. Agent nettoyant cosmétique doux selon la revendication 9, **caractérisé en ce qu'**il contient au moins un polymère cationique, sélectionné parmi les dérivés de cellulose cationiques, les dérivés de cellulose cationiques modifiés de manière hydrophobe, et/ou les dérivés de guar cationiques, et la glycérol.

11. Agent nettoyant cosmétique doux selon l'une quelconque des revendications 1 à 10, **caractérisé en ce qu'**il contient en plus au moins une substance amère, qui comporte une masse molaire d'au moins 250 g/mol, et qui est soluble dans l'agent nettoyant à 20 °C à au moins 10 mg/l.

12. Agent nettoyant cosmétique doux selon l'une quelconque des revendications 1 à 11, **caractérisé en ce qu'**il contient - rapporté à son poids total,
a) de 1,1 à 5 % en poids d'au moins un tensioactif anionique de la formule ci-dessus (I), dans laquelle le résidu R¹ représente un résidu alkyle linéaire ou ramifié, saturé ou insaturé avec 8 à 18 atomes de carbone, au moins un des résidus R² à R⁵ représente un résidu méthyle et les autres résidus représentent respectivement un atome d'hydrogène, et M+ représente un cation de métal alcalin ou un ion ammonium,
b) de 2,5 à 8,5 % en poids d'au moins un tensioactif amphotère et/ou zwittérionique d'une des formules générales mentionnées ci-dessus (iii), (v), (vi) ou (vii),
c) de 0,75 à 8 % en poids d'au moins un tensioactif non ionique et/ou un émulsifiant non ionique d'une des formules générales mentionnées ci-dessus (II), (III) ou RO-[G]ₓ,
d) au moins principe actif de conditionnement sélectionné parmi le groupe des hydrolysats de protéine, des polymères cationiques, des vitamines, des huiles végétales et de la glycérine, et
e) au moins une substance amère, qui comporte une masse molaire d'au moins 250 g/mol, et qui est soluble dans l'agent nettoyant à 20 °C à au moins 10 mg/l.

13. Agent nettoyant cosmétique doux selon l'une quelconque des revendications 1 à 12, **caractérisé en ce qu'**il présente une valeur de pH allant de 4 à 5,5, de préférence de 4,5 à 5,5.

14. Utilisation d'un agent nettoyant cosmétique doux selon l'une quelconque des revendications 1 à 13 en tant qu'agent nettoyant pour le visage sensible, en tant que shampoing pour bébés et/ou en tant que produit pour le bain des bébés.
